Europäisches Patentamt

**European Patent Office**

**Office européen des brevets**

(19)

(11) **EP 0 967 961 B1**

(12) **EUROPÄISCHE PATENTSCHRIFT**

(45) Veröffentlichungstag und Bekanntmachung des Hinweises auf die Patenterteilung:
**25.02.2004 Patentblatt 2004/09**

(21) Anmeldenummer: **98966275.4**

(22) Anmeldetag: **09.12.1998**

(51) Int Cl.⁷: **A61K 7/00**

(86) Internationale Anmeldenummer:
**PCT/EP1998/008017**

(87) Internationale Veröffentlichungsnummer:
**WO 1999/032067 (01.07.1999 Gazette 1999/26)**

(54) **VERFAHREN ZUR FORM- UND VOLUMENGEBUNG VON HUMANHAAREN**

METHOD FOR GIVING SHAPE AND VOLUME TO HUMAN HAIR

PROCEDE POUR DONNER UNE CERTAINE FORME ET DU VOLUME A DES CHEVEUX HUMAINS

(84) Benannte Vertragsstaaten:
**DE ES FR GB IT**

(30) Priorität: **19.12.1997 DE 19756654**

(43) Veröffentlichungstag der Anmeldung:
**05.01.2000 Patentblatt 2000/01**

(73) Patentinhaber: **Wella Aktiengesellschaft**
**64274 Darmstadt (DE)**

(72) Erfinder:
• **SCHONERT, Dieter**
**D-64354 Reinheim (DE)**
• **DANNECKER, Beate**
**D-64283 Darmstadt (DE)**
• **LANG, Günther**
**D-64354 Reinheim (DE)**
• **HOCH, Dieter**
**D-64319 Pfungstadt-Eich (DE)**
• **KALBACHER, Wilfried, K.**
**CEP-01424-000 Sao Paulo, SP (BR)**
• **MARKL, Johann**
**CEP-22441-030 Rio de Janeiro, RJ (BR)**

(56) Entgegenhaltungen:
**EP-A1- 0 443 356      EP-A1- 0 689 827**
**EP-A1- 0 880 916      US-A- 4 982 749**
**US-A- 5 570 708**

• **Database WPI on Epoque, week 9429, London: Derwent Publications Ltd., AN 94-238625, Klasse D21, XP002900458; & JP,A,06 172141 (TAKARA BELMONT KK), abstract.**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zum schonenden dauerhaften Verformen (Dauerwellen oder Entkräuseln) von menschlichen Haaren, bei dem man das Haarverformungsmittel vor dem Wickeln oder der Anwendung von anderen Befestigungskörpern oder dem Kämmen bei der Haarentkräuselung abspült.

Die Formgebung der Haare beim klassischen Dauerwellverfahren wird dadurch erzielt, daß vorgewaschene und damit gequollene, jedoch nicht vorreduzierte Haare durch Verwendung eines Formkörpers, insbesondere Wickler, unter Zug gebogen (Zugspannung) und mit Dauerwellreduhonsmittel behandelt werden. Das Dauerwellmittel läßt man in Abhängigkeit von der gewünschten Haarverformung 3 bis 30 Minuten auf das gewickelte Haar einwirken, bis eine ausreichende Umformung erzielt ist. Nach dem Einwirken des Dauerwellmittels werden Überschüsse an Reduktionsmittel mit Wasser oder unter Verwendung einer sauren Spülung entfernt. Trotz sorgfältigem Spülen verbleibt jedoch ein geringer Teil an Reduktionsmittel an der Haaroberfläche und im Haarinnern (näheres hierzu: vgl. SÖFW 123, S. 79 -83 1997). Nach dem Spülen werden die Cystin-Disulfidbindungen unter Verwendung eines Fixiermittels d.h. eines einen oxidierenden Wirkstoff enthaltenden Mittels, wieder geschlossen.

Beim klassischen Dauerwellverfahren wird die Einwirkdauer des reduktionsmittelhaltigen Veformungsmittels vom Friseur in der Regel durch Beurteilen der Umformungsstärke mittels mehrerer Probewickler bestimmt. Häufig ist der Friseur dabei unsicher und verlängert die Einwirkdauer, um eine ausreichende Verformung sicherzustellen. Eine zu lange Einwirkdauer hat jedoch zur Folge, daß das Haar, insbesondere in den Haartängen und Spitzen, überbehandelt wird. Man spricht vom Überkrausen des Haares. Dies äußert sich zum einen visuell, indem das Lockenbild in den Längen und Spitzen besonders kleinlockig erscheint (Krause) und zum zweiten sich der überkrauste. Haarbereich trotz starker Krause schlaff anfühlt und eine mangelnde Elastizität aufweist. Derartige Überkrausungseffekte führen zu einer irreversiblen, bleibenden Haarschädigung.

Aus der am 04.10.1956 bekanntgemachten deutschen Patentanmeldung G8775 IVa/30h ist ein Verfahren zur dauerhaften Verformung von Keratinfasem bekannt, bei dem die gewickelten und einer reaktiven reduzierenden Lösung ausgesetzten Keratinfasem nach einer üblicherweise für die Verformung noch nicht ausreichenden Einwirkungszeit mit Wasser ausgespüit und sodann zur Luftoxidation 2 bis 3,5 Stunden lang auf dem Wickler belassen werden. Die Anwendung eines chemischen Fixiermittels ist nicht erforderlich. Dieses sehr zeitaufwendige und für den Kunden lästige Verfahren hat sich beim Friseur nicht durchgesetzt.

Aus der eigenen DE-A- 3610 394 ist ein Verfahren zur Verformung von Haaren, bekannt, bei dem man das Haar mit einem Haarverformungshampoo vom pH 6,0 bis 7,5 , enthaltend 1,5 bis 12 Gewichtsprozent mindestens eines Tensids und 2 bis 15 Gewichtsprozent mindestens eines haarkeratinreduzierenden Wirkstoffes behandelt, mit Wasser spült, auf den Wickler wickelt, ggf. mit Wasser spült und sodann oxidativ nachbehandelt.

EP 0880916 beschreibt ein Verfahren zum dauerhaften Haarverformen. Das vorliegende Verfahren unterscheidet sich davon in der Reihenfolge der verschiedene Verfahrensschritte; in EP 0880916 wird das Haar zuerst aufgewickelt, anstatt nach der Zwischenbehandlung wie in der vorliegenden Anmeldung.

EP 0443356 offenbart ein Verfahren zur Erzeugung von Dauerwellen, das einen Reduktions-, einen Spül- und einen Reoxidationsschritt enthält (Beispiel 1), worin das Spülen nicht mit Wasser, sondern mit einer wäßrigen Natriumchlorid-Lösung durchgeführt wird.

Den derzeit üblichen Verformungsverfahren ist gemeinsam, daß die reduzierenden Dauerverformungsmittel über die gesamte, für die Haarverformung erforderliche Zeit in unverminderter Stärke auf das Haar einwirken und dadurch, insbesondere bei mehrfacher Haarverformung, zu irreversiblen Haarschädigungen, fachlich als "Überkrausung" bezeichnet, führen. Die negativen Folgen für das Haar sind mangelnde Elastizität und Sprungkraft, verminderte Reißfestigkeit, schlechte Frisierbarkeit und verminderter Glanz.

Zur Vermeidung der oben genannten Überkrausungseffekte wurde nach einem neuen Verfahren gesucht, mit denen die Einwirkdauer des chemischen Dauerwellmittels auf das Haar merklich reduziert, aber auch der Kontakt des Reduktionsmittes mit der Kopfhaut gegenüber den herkömmlichen Verfahren auf ein Minimum begrenzt werden kann. Dadurch soll die Haarstruktur weniger geschädigt werden. Zudem sollen nach dem Entfernen des Reduktionsmittels im Haarinnern verbleibende Rückstände an Thioverbindungen bei der Verformung mitgenutzt werden.

Es wurde nunmehr überraschend gefunden, daß ein Verfahren zur Form- und Volumengebung von Haaren, dadurch gekennzeichnet, daß man

a) auf das Haar ein alkalisches Dauerverformungsmittel vom pH 7,6 bis 11 auf der Basis einer haarkeratinreduzierenden Substanz aufbringt,

b) das Dauerverformungsmittel 5 bis 30 Minuten lang bei Raumtemperatur oder 2 bis 20 Minuten lang bei erhöhter Temperatur auf das Haar einwirken läßt,

c) gegebenenfalls mit Wasser spült,

d) das Haar mit einem sauren wäßrigen Zwischenbehandlungsmittel vom pH 2 bis 6,5 auf der Basis eines Salzes, eines Betains, einer aliphatischen organischen Säure und/oder einer aliphatischen Aminosäure behandelt,

e) gegebenenfalls überschüssige Flüssigkeit vom Haar abtupft,

f) das Haar auf Wickler wickelt, andere Formkörper oder Befestigungsmittel anwendet oder dem Haar in sonstiger Weise eine neue Form gibt,

g) das Haar bis zu 30 Minuten lang bei Raumtemperatur oder bis zu 20 Minuten lang bei erhöhter Temperatur ruhen läßt,

h) das Haar nach dem Schritt e) oder nach dem Schritt g) mit einem Fixiermittel auf der Basis eines Oxidationsmittels behandelt,

i) falls Wickler oder andere Formkörper oder Befestigungsmittel verwendet wurden, diese entfernt und

j) das Fixiermittel mit Wasser aus dem Haar ausspült oder mit einem Shampoo auswäscht und das Haar zur gewünschten Frisur kämmt,

die gestellte Aufgabe löst.

Bei dem erfindungsgemäßen Verfahren wird das, vorzugsweise trockene, Haar mit einem flüssigen, cremeartigen oder gelartigen Haardauerverformungsmittels auf der Basis einer haarkeratinreduzierenden Substanz in einer für die Haarverformung ausreichenden Menge, vorzugsweise 60 bis 120 Gramm, behandelt. Hierbei eignen sich insbesondere cremeartige und gelartige Haardauerverformungsmittel, die am trockenen Haar gut haften. Das Haardauerverformungsmittel gelangt dabei in unverdünnter Form auf das Haar bzw. in das Haarinnere. Die Reduktionsphase kann nach einer definierten, möglichst kurzen Einwirkungszeit, ohne hinderliche Formkörper auf dem ganzen Kopf, abgebrochen werden, indem mit einem sauren Zwischenbehandlungsrnittel in Form einer Spülung über den ganzen Kopf gespült wird . Hierbei nimmt das reduzierte Haar Wasser auf. Der nach dem Ausspülen verbleibende Anteil an Reduktionsmittelresten im Haarinnern wird gezielt für die Form- und Volumengebung genutzt. Der Verformungsprozeß wird durch zwei Faktoren begünstigt: Das Haar wird nicht nur vorgequollen, sondern bereits vorreduziert in die gewünschte Form gebracht.

Die Form- bzw. Volumengebung nach dem hier beschriebenen Verfahren kann, in Abhängigkeit von der Haarstruktur, in einem (reduzieren, spülen, fixieren und dann wickeln) oder in zwei Schritten (reduzieren, spülen, wickeln und dann fixieren) durchgeführt werden. Somit bietet das neue Verfahren für Friseure und Kunden den Vorteil, daß auf individuelle Wünsche und besonders auf die Haarstruktur eingegangen werden kann, indem in einem Einstufenprozeß reduziert und fixiert wird oder in einem Zweistufenprozeß zunächst reduziert und dann fixiert wird.

Überraschend wurde gefunden, daß im Falle des erfindungsgemäßen Verfahrens bei gleicher Einwirkungsdauer, insbesondere beim Anwenden verdickter Haardauerverformungsmittel (Creme, Gel, Paste), gegenüber einem flüssigen Haardauerverformungsmittel sich verstärkte Verformungseffekte partiell bzw. komplett dadurch erzielen lassen, daß an Stelle von feuchten Haaren wie beim klassischen Wellverfahren trockene Haare (ungewaschene, trockene Haare oder gewaschene und getrocknete Haare) verwendet werden.

Für die Haarentkräuselung kann dem Haar eine neue Form, anstatt mit (großen) Wicklern, auch durch Kämmen gegeben werden. Diese Ausführungsform des erfindungsgemäßen Verfahrens ist dadurch gekennzeichnet, daß man

A) auf das Haar ein creme-, gel- oder pastenförmiges alkalisches Dauerverformungsmittel vom pH 7,6 bis 11 auf der Basis einer haarkeratinreduzierenden Substanz aufbringt,

B). das Darverformungsmittel 10 bis 30 Minuten lang bei Raumtemperatur oder 5 bis 20 Minuten lang bei erhöhter Temperatur auf das Haar einwirken läßt,

C) das Haar mit Wasser spült,

D) auf das Haar ein saures wäßriges, creme- gel- oder pastenförmiges Zwischenbehandlungsmittel vom pH 2 bis 6,5 auf der Basis eines Salzes, eines Betains, einer aliphatischen organischen Säure und/oder einer aliphatischen Aminosäure aufbringt,

E) das Haar (vorzugsweise 1 bis 3 mal) durchkämmt,

F) das Haar bis zu 30 Minuten lang bei Raumtemperatur oder bis zu 20 Minuten lang bei erhöhter Temperatur ruhen läßt,

G) das Haar mit einem Fixiermittel auf der Basis eines Oxidationsmittels behandelt,

H) das Fixiermittel mit Wasser aus dem Haar ausspült oder mit einem Shampoo auswascht und das Haar zur gewünschten Frisur kämmt.

Als haakeratinreduzierende Substanz sind alle üblichen derartigen Stoffe geeignet. Es können insbesondere Thioglykolsäure, Thioglykolsäureamide, Thiomilchsäure, 3-Mercapto-propionsäure, Cystein, Cysteamin, Alkyl- oder Acylcysteamine oder die Salze dieser Verbindungen, auch im Gemisch miteinander, eingesetzt werden.

Die in dem Haardauerverformungsmittel enthaltenen haarkeratinreduzierenden Wirkstoffe werden in dem Mittel zur dauerhaften Haarverformung bevorzugt in einer Menge von 2 bis 20 Gewichtsprozent, besonders bevorzugt in einer Menge von 4 bis 13 Gewichtsprozent und ganz besonders bevorzugt in einer Menge von 6 bis 10 Gewichtsprozent eingesetzt.

Neben den genannten oder anderen, an sich bekannten Reduktionsmitteln, enthalten sie üblicherweise ein Alkalisierungsmittel, dessen Konzentration von Art und Menge des haarkeratinreduzierenden Wirkstoffs abhängt. Die Dauerverformungsmittel können insbesondere einen pH-Wert von 3,5 bis 10 aufweisen; sie sind bevorzugt alkalisch eingestellt und enthalten dann als Alkalisierungsmittel bzw. als Mittel zur Einstellung des pH-Wertes insbesondere Ammoniak, Natronlauge oder wasserlösliche, physiologisch verträgliche organische Basen, wie z.B. Di- oder Triethanolamin. Bevorzugtes Alkalisierungsmittel ist Ammoniak, wobei, je nach haarkeratinreduzierendem Wirkstoff, die Einstellung des pH-Wertes auf den alkalischen Bereich zwischen 7,1 und 11, vorzugsweise 7,5 bis 9,5, erfolgt.

Das Haardauerverformungsmittel kann sowohl in Form einer wäßrigen Lösung oder einer Emulsion als auch in verdickter Form auf wäßriger Basis, insbesondere als Creme, Gel oder Paste vorliegen. Ist das Haardauerverformungsmittel als Creme, Gel oder Paste formuliert, so ist liegt die bevorzugte Viskosität im Bereich von 200 bis 6 000 mPa·s, besonders bevorzugt 1 000 bis 5 000 mPa·s, bei 25°C und einem Schergefälle von 12,9 s$^{-1}$.

Selbstverständlich kann das Haardauerverformungsmittel alle für derartige Mittel üblichen und bekannten Zusatzstoffe, zum Beispiel Verdickungsmittel, wie beispielsweise, Bentonit, Fettsäuren, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; Alkohole, wie zum Beispiel Ethanol, Propanol, Isopropanol und Glycerin; Zucker wie z. B. D-Glucose; Lösungsvermittler, Stabilisatoren, Puffersubstanzen, Parfümöle, Farbstoffe sowie haarkonditionierende und haarpflegende Bestandteile, wie zum Beispiel kationische Polymere, z. B. Polydimethyldiallylammoniumchlorid (CTFA Polyquaternium-6), Polydimethyl-aminoethylmethacrylat (zu 75% quaternisiert mit Diethylsulfat CTFA Polyquaternium-11), CTFA Polyquaternium-4, CTFA Polyquaternium-5, CTFA Polyquaternium-7, CTFA Polyquaternium-9, CTFA Polyquaternium-10. CTFA Polyquaternium-14, CTFA Polyquaternium-16, CTFA Polyquaternium-22, Lanolinderivate, Cholesterin, Pantothensäure und Betain, enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0,01 bis 1 Gewichtsprozent, die Puffersubstanzen in einer Menge von insgesamt 0,1 bis 10 Gewichtsprozent, Zucker, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 1 bis 30 Gewichtsprozent sowie zur Vermeidung einer Überkrausung der Haare Dithioverbindungen, beispielsweise Dithiodiglykolsäure, Dithiomilchsäure, die Dithiole der genannten Verbindungen oder die jeweiligen Salze, zugesetzt werden.

Nach einer für die dauerhafte Verformung des Haares noch nicht ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (vorzugsweise 7 bis 15 Minuten) bei Raumtemperatur und 2 bis 20 Minuten (vorzugsweise 5 bis 10 Minuten bei erhöhter Temperatur) beträgt, werden die Haare mit einer wäßrigen sauren Zwischenspülung gemäß Verfahrensschritt d) gespült oder für die Haarentkräuselung zunächst mit Wasser gespült und dann mit einem wäßrigen sauren, creme-, gel- oder pastenförmigen Zwischenbehandlungsmittel gemäß Verfahrensschritt D) behandelt. Wird ein creme-, gel- oder pastenförmiges Zwischenbehandlungsmittel verwendet, so ist dessen bevorzugte Viskosität im Bereich von 1 000 bis 500 000 mPa·s, besonders bevorzugt 4 000 bis 50 000 mPa·s, bei 25°C und einem Schergefälle von 12,9 s$^{-1}$.

Das wäßrige Zwischenbehandlungsmittel ist sauer eingestellt und enthält bevorzugt eine aliphatische organische Säure, insbesondere eine physiologisch verträgliche Säure ausgewählt aus Zitronensäure, Weinsäure, Milchsäure, Essigsäure, Glyoxylsäure, Maleinsäure und Fumarsäure. Diese Säuren können allein oder im Gemisch miteinander, je, nach Menge des für die Behandlung vorgesehenen wäßrigen Zwischenbehandlung, in einer Menge von 0,05 bis 3 Gew.% enthalten sein, wobei der pH-Wert bevorzugt 2,0 bis 5,0 und besonders bevorzugt 2,5 bis 4,5 beträgt. Das gegebenenfalls enthaltene Salz kann, neben bekannten physiologisch verträglichen anorganischen und organischen Salzen, z. B. NaCl, Na$_2$SO$_4$, auch das Salz der aliphatischen organischen Säure oder der aliphatischen Aminosäure sein.

Auch das Zwischenbehandlungsmittel bzw. die Zwischenspülung kann haarpflegende und haarkonditionierende Komponenten, z. B. kationische Polymere, wie sie beispielsweise vorstehend als Bestandteil des Dauerverformungsmittels genannt wurden, oder Lanolin, Lecithin, Glycerin, Allantoin, Purcellinöl, Silikonöl, Walrat, Wollwachs, Paraffinöl, Bienenwachs, Harnstoff, niedrigsiedende Isoparaffine, Eiweiß-Fettsäurekondensat, Keratinhydrolysat, Betain, Cholesterin oder Pantothensäure enthalten. Die haarkonditionierende Komponente ist in dem wäßrigen . Zwischenbehandlungsmittel allein oder im Gemisch in einer Menge von 0,1 bis 5 Gew.% enthalten. Das wäßrige Zwischenbehandlungsmittel kann auch vorteilhaft Salze mehrwertiger Metalle, insbesondere des Magnesiums, Calciums oder des Aluminiums, z. B. MgCl$_2$, AlCl$_3$ und MgSO$_4$, in einer Menge von 0,5 bis 5 Gew.% enthalten.

Im Falle der Verwendung einer sauren wäßrigen Zwischenspülung als Zwischenbehandlungsmittel wird nun, falls erforderlich, überschüssige Spülflüssigkeit von den Wickeln abgetupft.

Das gespülte Haar wird bei einer bevorzugten Ausführungsform des Verfahrens nun in einzelne Strähnen aufgeteilt und auf Wickler mit einem Durchmesser von 5 bis 30 Millimetern, bevorzugt 5 bis 15 Millimeter, gewickelt. Es können auch beliebige andere Formkörper oder Befestigungsmittel wie gewellte Folien, Spiralwickler oder Clipse, z. B. für Papilloten, angewendet werden.

Im Falle einer Haarentkräuselung (ohne Befestigungsmittel) nach der Methode des Kämmens wird das Haar während einer Einwirkungszeit von 10 bis 30 Minuten bei Raumtemperatur oder bis zu 20 Minuten, vorzugsweise 8 bis 12 Minuten, bei erhöhter Temperatur, 1 bis 3 mal mit einem grobzinkigen Kamm durchgekämmt und sodann oxidativ nachbehandelt ("fixiert").

Im Falle der Verwendung von Befestigungsmitteln wird Nach einer Ruhezeit des Haares auf dem Befestigungsmittel von bis zu 30 Minuten, vorzugsweise 10 bis 20 Minuten, bei Raumtemperatur oder bis zu 20 Minuten, vorzugsweise 8 bis 12 Minuten, bei erhöhter Temperatur, das Haar oxidativ nachbehandelt.

Das Fixiermittel wird, je nach Haarfülle, vorzugsweise in einer Menge von 80 bis 100 Gramm, angewendet. Für die oxidative Nachbehandlung im aufgewickelten oder nicht gewickelten Zustand des Haares kann jedes beliebige, für eine derartige Behandlung geeignete Fixiermittel verwendet werden. Beispiele für in solchen Fixiermitteln verwendbare Oxidationsmittel sind Kalium- und Natriumbromat, Natriumperborat, Harnstoffperoxid und Wasserstoffperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das bevorzugte Fixiermittel ist eine 0,5 bis 5%-ige wäßrige Lösung von Wasserstoffperoxid. Das Mittel für die oxidative Nachbehandlung kann selbstverständlich weitere Stoffe, wie zum Beispiel Netzmittel, Pflegestoffe wie kationische Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gew.% in dem Fixiermittel enthalten sein. Liegt das Fixiermittel als Creme, Gel oder Paste vor, so liegt seine bevorzugte Viskosität im Bereich von 1 000 bis 6 000 mPa·s, besonders bevorzugt im Bereich von 1 000 bis 3 000 mPa·s, bei 25°C und einem Schergefälle von 12,9 s$^{-1}$.

In einer alternativen Verfahrensvariante unter Verwendung von Befestigungsmifteln wird das Fixiermittel direkt nach der vorstehend beschriebenen Spülung des Haares mit einer sauren wäßrigen Zwischenspülung auf das Haar aufgetragen und dann erst erfolgt das Anbringen der Befestigungsmittel, z. B. der Wickler. In diesem Fall kann eine weitere Anwendung eines Fixiermittels nach dem Wickeln und dem Ruhenlassen (Schritt f) ) entfallen.

Anschließend werden die Befestigungsmittel bzw. Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Es hat sich gezeigt, daß durch Anwendung des erfindungsgemäßen Verfahrens ein Überkrausungseffekt und die damit verbundene Haarschädigung weitgehend vermieden wird.

Unter "erhöhter Temperatur" wird eine Temperatur von 35 bis 60 Grad Celsius, vorugsweise 40 bis 50 Grad Celsius, verstanden. Zur Temperaturerhöhung kann beispielsweise eine Trockenhaube, mit oder ohne Verwendung einer Abdeckhaube, oder aber ein Infrarotstrahlungsgerät angewandt werden.

Die nachfolgenden Ausführungsbeispiele dienen der näheren Illustration der Erfindung.

**Beispiel-1 Verfahren zur Dauerwellung**

Auf durch Farbbehandlung vorgeschädigtes, 15 cm langes trockenes Haar wird (ohne es vorher mit einem Shampoo zu waschen) ein flüssiges alkalisches Dauerwellmittel vom pH = 8,0 der nachfolgenden Zusammensetzung gleichmäßig verteilt.

| | |
|---|---|
| 15,2 Gew.% | Ammoniumthioglykolat, 70%ig |
| 3,5 Gew.% | Ammoniumhydrogencarbonat |
| 1,0 Gew.% | Ammoniak, 25%ig |
| 0,8 Gew.% | Polydimethyldiallylammoniumchlorid |
| 1,5 Gew.% | Harnstoff |
| 78,0 Gew.% | Wasser, vollentsalzt |
| 100,0 Gew.% | |

Das Dauerwellmittel weist eine Viskosität von 2 mPa·s bei 25° C und einem Schergefälle von 12,9 s$^{-1}$ auf.
Nach einer Einwirkungszeit von 8 Minuten bei einer, durch Anwendung eines Infrarot-Strahlungsgerätes erhöhten Temperatur von 40°C, werden die Haare mit 500 ml einer sauren wäßrigen Zwischenspülung vom pH = 2,6 der Zusammensetzung.

| | |
|---|---|
| 0,4 Gew.% | Milchsäure |
| 2,8 Gew.% | Magnesiumsulfat |
| 2,0 Gew.% | Betain |
| 94.8 Gew.% | Wasser |
| 100,0 Gew.% | |

(die Zwischenspülung weist eine Viskosität von 2 mPa·s bei einem Schergefälle von 12,9 s$^{-1}$ und 25° C auf) gespült.
Nach dem Abtupfen der überschüssigen Flüssigkeit wird das Haar auf Wickler mit einem Durchmesser von 6 bis 8 mm gewickelt.
Nach einer Ruhezeit von 10 Minuten bei einer, durch Anwendung eines Infrarot-Strahlungsgerätes erhöhten Temperatur von 40°C, werden die Wickel 150 ml einer 2,5 %igen wäßrigen Wasserstoffperoxidlösung oxidativ nachbehandelt. Nach Entfernung der Wickler werden die Haare mit Wasser gespült und anschließend getrocknet.
Als Ergebnis dieser Behandlung zeigte das Haar ein großzügiges Lockenbild, mehr Volumen, guten Griff und gute Naßkämmbarkeit.

**Beispiel 2**

Auf normales, nicht vorgeschädigtes 15 cm langes trockenes Haar wird (ohne es vorher mit einem Shampoo zu waschen) das alkalische Dauerwellmittel vom pH = 8,6 der nachfolgenden Zusammensetzung gleichmäßig aufgetragen:

| | |
|---|---|
| 17,0 Gew.% | Ammoniumthioglykolat, 70%-ig |
| 3,4 Gew.% | Ammoniumthiolactat, 70%-ig |
| 1.8 Gew.% | Ammoniak, 25%-ige wäßrige Lösung |
| 0,5 Gew.% | Polydimethyldiallylammoniumchlorid |
| 3,0 Gew.% | Harnstoff |
| 1,5 Gew.% | 1,2-Propylenglykol |
| 72,8 Gew.% | Wasser |
| 100,0 Gew.% | |

Nach einer Einwirkungszeit von 8 Minuten bei einer, durch Anwendung eines Infrarot-Strahlungsgerätes erhöhten Temperatur von 40°C, wird das Haar mit 500 ml einer sauren wäßrigen Zwischenspülung vom pH = 2,8 der Zusammensetzung

| | |
|---|---|
| 0,5 Gew.% | Zitronensäure |

(fortgesetzt)

| | |
|---|---|
| 0,5 Gew.% | Keratinhydrolysat, quaternisiert |
| 0,6 Gew.% | Cetyltrimethylammoniumchlorid |
| 1,0 Gew.% | Dimethylcarboxymethyl-kokosfettsäureamidoammoniumbetain (Rewoteric® AM B 14) |
| 97,4 Gew.% | Wasser |
| 100,0 Gew.% | |

gespült.

Die Haare werden sodann wie in Beispiel 1 weiterbehandelt.

Als Ergebnis dieser Behandlung zeigte das Haar ein großzügiges Lockenbild, mehr Volumen, guten Griff und gute Naßkämmbarkeit.

**Beispiel 3**

Auf normales, nicht vorgeschädigtes 15 cm langes trockenes Haar wird (ohne es vorher mit einem Shampoo zu waschen) die alkalische Dauerwellcreme vom pH = 8,3 der nachfolgenden Zusammensetzung gleichmäßig aufgetragen:

| | |
|---|---|
| 11,0 Gew.-% | Ammoniumthioglykolat, 70%ig |
| 0,4 Gew.-% | Ammoniak 25%ig |
| 1,0 Gew.-% | Ammoniumhydrogencarbonat |
| 0,2 Gew.-% | Tetrasodium Etidronate (Turpinal® 4 NL) |
| 5,8 Gew.-% | Paraffinium perliquidum (Mineralöl) |
| 8,2 Gew.-% | Stearylalkohol |
| 1,1 Gew.-% | Stearinsäure |
| 5,0 Gew.-% | Laurylalkoholpolyglykolether mit 23 EO |
| 0,4 Gew.-% | Cellulose Gum (Tylose® CB 4000) |
| 66.9 Gew.-% | Wasser |
| 100,0 Gew.% | |

Viskosität: 1 500 bis 4 500 mPa·s (SV-DIN; Stufe 5; 25°C; Schergefälle 12,9 s$^{-1}$)

Die Haare werden sodann wie in Beispiel 1 weiterbehandelt.

Als Ergebnis dieser Behandlung zeigte das Haar ein großzügiges Lockenbild, mehr Volumen, guten Griff und gute Naßkämmbarkeit.

**Beispiel 4**

Auf durch Farbbehandlung vorgeschädigtes, 18 cm langes trockenes Haar wird (ohne es vorher mit einem Shampoo zu waschen) ein alkalisches Dauerwellgel vom pH = 8,0 der nachfolgenden Zusammensetzung gleichmäßig verteilt:

| | |
|---|---|
| 10,0 Gew.-% | Ammoniumthioglykolat 70%ig |
| 3,0 Gew.-% | Cystein |
| 0,3 Gew.-% | Ammoniak 25%ig (pH = 8,0) |
| 20,0 Gew.-% | Polyoxyethylen/Polyoxypropylen-Blockpolymer (CTFA: Poloxamer 407) |
| 0,2 Gew.-% | Tetrasodium Etidronate (Turpinal® 4 NL) |
| 66.5 Gew.-% | Wasser |
| 100,0 Gew.-% | |

Viskosität: 1 000 bis 3 500 mPa·s (SV-DIN; Stufe 5; 25°C; Schergefälle 12,9 s$^{-1}$)

Nach einer Einwirkungszeit von 10 Minuten bei einer, durch Anwendung eines Infrarot-Strahlungsgerätes erhöhten Temperatur von 40°C, wird das Haar mit 500 ml einer sauren wäßrigen Zwischenspülung vom pH = 2,8 der Zusammensetzung

| | |
|---|---|
| 0,5 Gew.% | Zitronensäure |
| 0,5 Gew.% | Keratinhydrolysat, quaternisiert |
| 0,6 Gew.% | Cetyltrimethylammoniumchlorid |
| 1,0 Gew.% | Dimethylcarboxymethyl-kokosfettsäureamidoammoniumbetain (Rewoteric® AM B 14) |
| 97.4 Gew.% | Wasser |
| 100,0 Gew.% | |

(die Zwischenspülung weist eine Viskosität von 2 mPa·s bei Schergefälle 12,9 s$^{-1}$ und 25° C auf) gespült. Die Haare werden sodann wie in Beispiel 1 weiterbehandelt.

Als Ergebnis dieser Behandlung zeigte das Haar ein großzügiges Lockenbild, mehr Volumen, guten Griff und gute Naßkämmbarkeit.

**Beispiel 5**

Auf durch Farbbehandlung vorgeschädigtes, 15 cm langes trockenes Haar wird (ohne es vorher mit einem Shampoo zu waschen) eine flüssige alkalische Dauerwellcreme vom pH = 8,4 der nachfolgenden Zusammensetzung gleichmäßig verteilt.

| | |
|---|---|
| 7,0 Gew.% | Thioglykolsäure, 99%ig |
| 5,0 Gew.% | Ammoniak, 25%ig |
| 4,0 Gew.% | Ammoniumhydrogencarbonat |
| 3,0 Gew.% | Paraffinium perliquidum (Mineralöl) |
| 2,0 Gew.% | Stearinsäure |
| 9,0 Gew.% | Stearylalkohol |
| 4.0 Gew.% | Cetylalkoholpolyethylenglykolether (25 EO) |
| 1,0 Gew.% | Parfümöl |
| 65,0 Gew.% | Wasser |
| 100,0 Gew.% | |

Viskosität: 1 500 bis 4 000 mPa·s (SV-DIN; Stufe 5; 25°; Schergefälle 12,9 s$^{-1}$)

Nach einer Einwirkungszeit von 15 Minuten bei einer, durch Anwendung eines Infrarot-Strahlungsgerätes erhöhten Temperatur von 40°C, werden die Haare mit 500 ml einer sauren wäßrigen Zwischenspülung vom pH = 2,6 der Zusammensetzung

| | |
|---|---|
| 0,4 Gew.% | Milchsäure |
| 2,8 Gew.% | Magnesiumsulfat |
| 2,0 Gew.% | Betain |
| 94,8. Gew.% | Wasser |
| 100,0 Gew.% | |

(die Zwischenspülung weist eine Viskosität von 2 mPa·s bei Schergefälle 12,9 s$^{-1}$ und 25°C auf) gespült.

Nach dem Abtupfen der überschüssigen Flüssigkeit trägt man auf das Haar eine Cremefixierung vom pH = 2,5 der nachfolgenden Zusammensetzung auf:

| | |
|---|---|
| 3,00 Gew.% | Wasserstoffperoxid |
| 9,00 Gew.% | Stearylalkohol |
| 2,50 Gew% | Cetylalkoholpolyethylengtykolether (25 EO) |
| 9,00 Gew.% | Paraffinium perliquidum (Mineralöl) |
| 2,00 Gew.% | Pentandiol |
| 1,00 Gew.% | Kaliumhydrogenphosphat |
| 0,25 Gew.% | Phosphorsäure, 85%ig |
| 73,25 Gew.% | Wasser |
| 100,00 Gew.% | |

Viskositat: 1 500 bis 4 000 mPa · s (SV-DIN; Stufe 5; 25°C, Schergefälle 12,9 s$^{-1}$)

Nun wird das Haar auf Wickler mit einem Durchmesser von 6 bis 8 mm gewickelt.

Nach einer Ruhezeit von 10 Minuten bei einer, durch Anwendung eines Infrarot-Strahlungsgerätes erhöhten Temperatur von 40°C, werden die Wickler aus dem Haar entfernt. Nach Entfernung der Wickler werden die Haare mit Wasser gespült un anschließend getrocknet.

Als Ergebnis dieser Behandlung zeigte das Haar ein großzügiges Lockenbild, mehr Volumen, guten Griff und gute Naßkämmbarkeit.

**Beispiel 6 Verfahren zur Haarentkräuselung**

Auf naturgekraustes, 18 cm langes trockenes Haar wird (ohne es vorher mit einem Shampoo zu waschen) 80 g eines cremeförmigen alkalischen Dauerverformungsmittels vom pH = 9,2 der nachfolgenden Zusammensetzung gleichmäßig verteilt.

| | |
|---|---|
| 9,0 Gew.% | Thioglykolsäure, 99%ig |
| 9,7 Gew.% | Ammoniak, 25%ig |
| 10,0 Gew.% | Gemisch aus Cetylalkohol und Stearylalkohol |
| 2,0 Gew.% | Cetylalkoholpolyethytenglykolether (25 EO) |
| 0,5 Gew.% | Parfümöl |
| 68.8 Gew.% | Wasser |
| 100,0 Gew.% | |

(Viskosität: 3 500 mPa·s bei 25°C und einem Schergefälle von 12,9 s$^{-1}$)

Nach einer Einwirkungszeit von 15 Minuten bei Raumtemperatur wird das Haar mit Wasser ausgespült und die überschüssige Feuchtigkeit mit einem Handtuch abgetupft. Sodann werden 80 g eines sauren pastenförmigen Zwischenbehandlungsmittels vom pH = 2,2 der Zusammensetzung

| | |
|---|---|
| 2,5 Gew.% | Milchsäure, 80%ig |
| 2,0 Gew.% | Magnesiumsulfat |
| 15,0 Gew.% | Gemisch aus Cetylalkohol und Stearylalkohol |
| 2,0 Gew.% | Cetylalkoholpolyethylenglykolether (25 EO) |
| 15,0 Gew.% | Bentonit |
| 0,5 Gew.% | Parfümöl |
| 63.0 Gew.% | Wasser |
| 100,0 Gew.% | |

(Viskosität: 26 000 mPa·s bei 25°C und einem Schergefälle von 12,9 s$^{-1}$) gleichmäßig auf das Haar aufgetragen und das Haar mit der Paste mit einem großzinkigem Kamm 3 mal durchgekämmt.

Nach dem Ablauf der Einwirkungszeit von 10 Minuten trägt man auf das Haar eine Cremefixierung vom pH = 2,3 der nachfolgenden Zusammensetzung auf und massiert diese in das Haar ein.

| | |
|---|---|
| 2,5 Gew.% | Wasserstoffperoxid |
| 7,0 Gew.% | Gemisch aus Cetylalkohol und Stearytalkohol |
| 2,0 Gew% | Cetylalkoholpolyethylenglykolether (25 EO) |
| 3,0 Gew.% | Petrolatum |
| 0,2 Gew.% | Dinatriumphosphat |
| 0,5 Gew.% | Parfümöl |
| 0,2 Gew.% | Phosphorsäure, 75%ig |
| 84,6 Gew.% | Wasser |
| 100,0 Gew.% | |

(Viskosität: 2 000 mPa·s bei 25°C und einem Schergefälle von 12,9 s$^{-1}$)

Nach einer Ruhezeit von 10 Minuten bei Raumtemperatur werden die Haare mit Wasser gespült und anschließend getrocknet.

Als Ergebnis dieser Behandlung ist das Haar geglättet und die ursprünglich starke Krause wurde in eine großzügige Locke überführt, wie sie die heutige moderne Frisurengestaltung verlangt. Zudem ist das Haar in einem guten Zustand, der sich im gutem Griff und guter Naßkämmbarkeit manifestiert.

**Beispiel 7 Verfahren zur Haarentkräuselung**

Auf naturgekraustes, 15 cm langes trockenes Haar werden (ohne es vorher mit einem Shampoo zu waschen) 70 g eines cremeförmigen alkalischen Dauerverformungsmittels vom pH = 9,4 der nachfolgenden Zusammensetzung gleichmäßig verteilt.

| | |
|---|---|
| 11,0 Gew. % | Thioglykolsäure, 99%ig |
| 13,2 Gew.% | Ammoniak, 25%ig |
| 5,0 Gew.% | Gemisch aus Cetylalkohol und Stearylalkohol |
| 1,0 Gew.% | Cetylalkoholpolyethylenglykolether (25 EO) |
| 0,5 Gew.% | Parfümöl |
| 69,3 Gew.% | Wasser |
| 100,0 Gew.% | |

(Viskosität: 1 500 mPa·s bei 25°C und einem Schergefälle von 12,9 s$^{-1}$)

Nach einer Einwirkungszeit von 12 Minuten bei Raumtemperatur wird das Haar mit Wasser ausgespült und die überschüssige Feuchtigkeit mit einem Handtuch entfernt. Sodann werden 70 g eines sauren pastenförmigen Zwischenbehandlungsmittels vom pH = 2,1 der Zusammensetzung

| | |
|---|---|
| 2,5 Gew.% | Milchsäure, 80%ig |
| 2,0 Gew.% | Magnesiumsulfat |
| 15,0 Gew.% | Gemisch aus Cetylalkohol und Stearylalkohol |
| 2,0 Gew.% | Cetylalkoholpolyethylenglykolether (25 EO) |
| 0,5 Gew.% | Parfümöl |
| 78,0 Gew.% | Wasser |
| 100,0 Gew.% | |

(Viskosität: 6 000 mPa · s bei 25°C und einem Schergefälle von 12,9 s$^{-1}$)

gleichmäßig auf das Haar aufgetragen und das Haar mit der Paste mit einem großzinkigem Kamm 2 mal durchgekämmt.

Nach dem Ablauf der Einwirkungszeit von 10 Minuten trägt man auf das Haar eine Cremefixierung vom pH = 4,3 der nachfolgenden Zusammensetzung auf:

| | |
|---|---|
| 11,0 Gew.% | Natriumbromat |
| 6,0 Gew.% | Gemisch aus Cetylalkohol und Stearylalkohol |
| 2,0 Gew% | Cetylalkoholpolyethylenglykolether (25 EO) |
| 5,0 Gew.% | Petrolatum |
| 0,5 Gew.% | Natriumphosphat |
| 0,5 Gew.% | kationisches Polymer (CTFA: Polyquatemium-6) |
| 0,5 Gew.% | Parfüm |
| 74.5 Gew.% | Wasser |
| 100.0 Gew.% | |

(Viskosität: 2 600 mPa·s bei 25°C und einem Schergefälle von 12,9 s$^{-1}$)

Nach einer Ruhezeit von 10 Minuten bei Raumtemperatur werden die Haare mit Wasser gespült und anschließend getrocknet.

Als Ergebnis dieser Behandlung ist das Haar geglättet und die ursprünglich starke Krause wurde in eine großzügige Locke überführt, wie sie die heutige moderne Frisurengestaltung verlangt. Zudem ist das Haar in einem guten Zustand, der sich im gutem Griff und guter Naßkämmbarkeit manifestiert.

**Patentansprüche**

1. Verfahren zur Form- und Volumengebung von Haaren, **dadurch gekennzeichnet, daß** man

   a) auf das Haar ein alkalisches Dauerverformungsmittel vom pH 7,6 bis 11 auf der Basis einer haarkeratinreduzierenden Substanz aufbringt,

   b) das Dauerverformungsmittel 5 bis 30 Minuten lang bei Raumtemperatur oder 2 bis 20 Minuten lang bei erhöhter Temperatur auf das Haar ein wirken läßt,

   c) gegebenenfalls mit Wasser spült,

   d) das Haar mit einem sauren wäßrigen Zwischenbehandlungsmittel vom pH 2 bis 6,5 auf der Basis eines Salzes, eines Betains, einer aliphatischen organische Säure und/oder einer aliphatischen Aminosäure behandelt,

   e) gegebenenfalls überschüssige Flüssigkeit vom Haar abtupft,

   f) das Haar auf Wickler wickelt, andere Formkörper oder Befestigungsmittel anwendet oder dem Haar in sonstiger Weise eine neue Form gibt,

   g) das Haar bis zu 30 Minuten lang bei Raumtemperatur oder bis zu 20 Minuten lang bei erhöhter Temperatur ruhen läßt,

   h) das Haar nach dem Schritt e) oder nach dem Schritt g) mit einem Fixiermittel auf der Basis eines Oxidationsmittels behandelt,

   i) falls Wickler oder andere Formkörper oder Befestigungsmittel verwendet wurden, diese entfernt und

   j) das Fixiermittel mit Wasser aus dem Haar ausspült oder mit einem Shampoo auswäscht und das Haar zur gewünschten Frisur kämmt.

2. Verfahren nach Anspruch 1, **dadurch gekennzeichnet, daß** man ein Dauerverformungsmittel verwendet, indem die haarkeratin-reduzierende Substanz ausgewählt ist aus Thioglykolsäure, Cystein oder Thiomilchsäure oder dem Salz dieser Verbindungen.

3. Verfahren nach einem der Ansprüche 1 oder 2, **dadurch gekennzeichnet, daß** man das Dauerverformungsmittel bei Raumtemperatur 7 bis 15 Minuten lang einwirken läßt.

4. Verfahren nach einem der Ansprüche 1 bis 3, **dadurch gekennzeichnet, daß** man das Dauerverformungsmittel bei einer Temperatur von 40 bis 50 Grad Celsius 5 bis 10 Minuten lang einwirken läßt.

5. Verfahren nach einem der Ansprüche 1 bis 4, **dadurch gekennzeichnet, daß** das Dauerverformungsmittel als Gel, Creme oder Paste vorliegt.

6. Verfahren nach Anspruch 5, **dadurch gekennzeichnet, daß** man

   A) auf das Haar ein creme-, gel-, oder pastenförmiges alkalisches Dauerverformungsmittel- vom pH 7,6 bis 11 auf der Basis einer haarkeratinreduzierenden Substanz aufbringt,

   B) das Dauerverformungsmittel 10 bis 30 Minuten lang bei Raumtemperatur oder 5 bis 20 Minuten lang bei erhöhter Temperatur auf das Haar einwirken läßt,

   C) das Haar mit Wasser spült,

   D) auf das Haar ein saures wäßriges, creme- gel- oder pastenförmiges Zwischenbehandlungsmittel vom pH 2 bis 6,5 auf der Basis eines Salzes, eines Betains, einer aliphatischen organischen Säure und/oder einer aliphatischen Aminosäure aufbringt,

E) das Haar durchkämmt,

F) das Haar bis zu 30 Minuten lang bei Raumtemperatur oder bis zu 20 Minuten lang bei erhöhter Temperatur ruhen läßt,

G) das Haar mit einem Fixiermittel auf der Basis eines Oxidationsmittels behandelt,

H) das Fixiermittel mit Wasser aus dem Haar ausspült oder mit einem Shampoo auswäscht und das Haar zur gewünschten Frisur kämmt.

**7.** Verfahren nach einem der Ansprüche 5 oder 6, **dadurch gekennzeichnet, daß** man ein Dauerverformungsmittel verwendet, das eine Viskosität im Bereich von 200 bis 6 000 mPa bei 25°C und einem Schergefälle von 12,9 s$^{-1}$ aufweist.

**8.** Verfahren nach nach einem der Ansprüche 6 oder 7, **dadurch gekennzeichnet, daß** man ein Zwischenbehandlungsmittel verwendet, dessen Viskosität im Bereich von 4 000 bis 50 000 mPa·s bei 25°C und einem Schergefälle von 12,9 s$^{-1}$ liegt.

**9.** Verfahren nach einem der Ansprüche 1 bis 8, **dadurch gekennzeichnet, daß** man als Fixiermittel eine wäßrige Lösung von Wasserstoffperoxid verwendet.

**10.** Verfahren nach einem der Ansprüche 1 bis 9, **dadurch gekennzeichnet, daß** man es an trockenen, unmittelbar vor der Anwendung des Verfahrens nicht gewaschenen Haaren anwendet.

**Claims**

**1.** Method of shaping and volumising hair, **characterized in that**

a) an alkaline permanent shaping agent of pH 7.6 to 11 based on a hair keratin-reducing substance is applied to the hair,

b) the permanent shaping agent is left to act on the hair for 5 to 30 minutes at room temperature or for 2 to 20 minutes at increased temperature,

c) optional rinsing with water is carried out,

d) the hair is treated with an acidic aqueous intermediate treatment agent of pH 2 to 6.5 based on a salt, a betaine, an aliphatic organic acid and/or an aliphatic amino acid,

e) excess liquid is optionally dabbed from the hair,

f) the hair is wound onto rollers, other shaped bodies or fixing means are used or a new shape is given to the hair in another way,

g) the hair is left to rest for up to 30 minutes at room temperature or for up to 20 minutes at increased temperature,

h) the hair after step e) or after step g) is treated with a fixing agent based on an oxidising agent,

i) if rollers or other shaped bodies or fixing means have been used, these are removed and

j) the fixing agent is rinsed out of the hair with water or washed out of the hair using a shampoo and the hair is combed into the desired hairstyle.

**2.** Method according to Claim 1, **characterized in that** a permanent shaping agent is used in which the hair keratin-reducing substance is chosen from thioglycolic acid, cysteine or thiolactic acid or the salt of these compounds.

**3.** Method according to either Claim 1 or 2, **characterized in that** the permanent shaping agent is left to act at room temperature for 7 to 15 minutes.

**4.** Method according to one of Claims 1 to 3, **characterized in that** the permanent shaping agent is left to act at a temperature of from 40 to 50°C for 5 to 10 minutes.

**5.** Method according to one of Claims 1 to 4, **characterized in that** the permanent shaping agent is in the form of a gel, cream or paste.

**6.** Method according to Claim 5, **characterized in that**

A) a cream-, gel- or paste-like alkaline permanent shaping agent of pH 7.6 to 11 based on a hair keratin-reducing substance is applied to the hair,

B) the permanent shaping agent is left to act on the hair for 10 to 30 minutes at room temperature or for 5 to 20 minutes at increased temperature,

C) the hair is rinsed with water,

D) an acidic aqueous, cream-, gel- or paste-like intermediate treatment agent of pH 2 to 6.5 based on a salt, a betaine, an aliphatic organic acid and/or an aliphatic amino acid is applied to the hair,

E) the hair is combed through,

F) the hair is left to rest for up to 30 minutes at room temperature or for up to 20 minutes at increased temperature,

G) the hair is treated with a fixing agent based on an oxidising agent,

H) the fixing agent is rinsed out of the hair with water or is washed out of the hair using a shampoo and the hair is combed into the desired hairstyle.

**7.** The method according to Claims 5 or 6, **characterized in that** a permanent shaping agent is used which has a viscosity in the range from 200 to 6000 mPa at 25°C and a shear gradient of 12.9 s$^{-1}$.

**8.** Method according to Claim 6 or 7, **characterized in that** an intermediate treatment agent is used whose viscosity is in the range from 4000 to 50 000 mPa·s at 25°C and a shear gradient of 12.9 s$^{-1}$.

**9.** Method according to one of Claims 1 to 8, **characterized in that** the fixing agent used is an aqueous solution of hydrogen peroxide.

**10.** Method according to one of Claims 1 to 9, **characterized in that** it is used on dry hair which has not been washed directly prior to using the method.

**Revendications**

**1.** Procédé pour la mise en forme des cheveux et leur donner du volume, **caractérisé en ce que**

a) on applique sur les cheveux une composition alcaline de mise en forme permanente à pH 7,6 à 11, à base d'une substance réduisant la kératine des cheveux,
b) on laisse agir sur les cheveux la composition de mise en forme permanente pendant 5 à 30 minutes à la température ambiante ou pendant 2 à 20 minutes à une température élevée,
c) éventuellement on rince à l'eau,
d) on traite les cheveux par une composition aqueuse acide de traitement intermédiaire à pH 2 à 6,5, à base d'un sel, d'une bétaïne, d'un acide organique aliphatique et/ou d'un aminoacide aliphatique,
e) éventuellement on enlève le liquide en excès des cheveux,
f) on enroule les cheveux sur des rouleaux, on applique d'autres éléments de mise en forme ou moyens de fixage ou on donne d'une autre façon une nouvelle forme aux cheveux,

g) on laisse reposer les cheveux pendant jusqu'à 30 minutes à la température ambiante ou pendant jusqu'à 20 minutes à une température élevée,

h) on traite les cheveux, après l'étape e) ou après l'étape g) par un fixateur à base d'un oxydant,

i) si l'on a utilisé des rouleaux ou d'autres éléments de mise en forme ou moyens de fixation, on les retire et

j) on élimine par lavage avec un shampooing ou par rinçage à l'eau le fixateur des cheveux et on peigne les cheveux à la coiffure désirée.

2. Procédé selon la revendication 1, **caractérisé en ce qu'**on utilise une composition de mise en forme permanente en choisissant la substance réduisant la kératine des cheveux parmi l'acide thioglycolique, la cystéine et l'acide thiolactique et les sels de ces composés.

3. Procédé selon la revendication 1 ou 2, **caractérisé en ce qu'**on laisse agir la composition de mise en forme permanente pendant 7 à 15 minutes à la température ambiante.

4. Procédé selon l'une quelconque des revendications 1 à 3, **caractérisé en ce qu'**on laisse agir la composition de mise en forme permanente pendant 5 à 10 minutes à une température de 40 à 50°C.

5. Procédé selon l'une quelconque des revendications 1 à 4, **caractérisé en ce que** la composition de mise en forme permanente se trouve sous forme d'un gel, d'une crème ou d'une pâte.

6. Procédé selon la revendication 5, **caractérisé en ce que**

A) on applique sur les cheveux une composition alcaline de mise en forme permanente sous forme de crème, de gel ou de pâte, à pH 7,6 à 11, à base d'une substance réduisant la kératine des cheveux,

B) on laisse agir sur les cheveux la composition de mise en forme permanente pendant 10 à 30 minutes à la température ambiante ou pendant 5 à 20 minutes à une température élevée,

C) on rince les cheveux à l'eau,

D) on applique sur les cheveux une composition aqueuse acide de traitement intermédiaire sous forme de crème, de gel ou de pâte, à pH 2 à 6,5, à base d'un sel, d'une bétaïne, d'un acide organique aliphatique et/ou d'un aminoacide aliphatique,

E) on peigne complètement les cheveux,

F) on laisse reposer les cheveux pendant jusqu'à 30 minutes à la température ambiante ou pendant jusqu'à 20 minutes à une température élevée,

G) on traite les cheveux par un fixateur à base d'un oxydant,

H) on élimine par lavage avec un shampooing ou par rinçage à l'eau le fixateur des cheveux et on peigne les cheveux à la coiffure désirée.

7. Procédé selon la revendication 5 ou 6, **caractérisé en ce qu'**on utilise une composition de mise en forme permanente qui présente une viscosité dans la plage de 200 à 6 000 mPa.s à 25°C et une vitesse de cisaillement de 12,9 s$^{-1}$.

8. Procédé selon la revendication 6 ou 7, **caractérisé en ce qu'**on utilise une composition de traitement intermédiaire dont la viscosité se trouve dans la plage allant de 4 000 à 50 000 mPa.s à 25°C et à une vitesse de cisaillement de 12,9 s$^{-1}$.

9. Procédé selon l'une quelconque des revendications 1 à 8, **caractérisé en ce qu'**on utilise comme fixateur une solution aqueuse de peroxyde d'hydrogène.

10. Procédé selon l'une quelconque des revendications 1 à 9, **caractérisé en ce qu'**on l'utilise sur des cheveux secs non lavés immédiatement avant l'emploi du procédé.